# EUROPEAN PATENT SPECIFICATION

(11) **EP 2 913 329 B1**
(45) Date of publication and mention of the grant of the patent: **27.11.2019**
(21) Application number: 13848455.5
(22) Date of filing: 24.10.2013
(51) Int. Cl.: C07D 307/77, C07D 405/06, C07B 39/00

(54) **METHOD FOR MANUFACTURING DIFLUORO LACTONE COMPOUND**
VERFAHREN ZUR HERSTELLUNG EINER DIFLUORLACTONVERBINDUNG
PROCÉDÉ DE FABRICATION D'UN COMPOSÉ LACTONE DIFLUORÉ

(30) Priority: 26.10.2012 JP 2012236261
(43) Date of publication of application: 02.09.2015
(73) Proprietor: AGC Inc., Tokyo 100-8405 (JP)
(72) Inventor: ISHIBASHI, Yuichiro, Tokyo 100-8405 (JP); MATSUMURA, Yasushi, Tokyo 100-8405 (JP)
(74) Representative: Müller-Boré & Partner Patentanwälte PartG mbB
(86) International application number: PCT/JP2013/078871
(87) International publication number: WO 2014/065382

(56) References cited:
- WO-A1-2010/029925
- WO-A1-2011/049126
- WO-A1-2011/111714
- WO-A2-2008/071587
- WO-A2-2011/003018
- JP-A- 2012 001 537
- YANA CEN ET AL: "Efficient Syntheses of Clofarabine and Gemcitabine From 2-Deoxyribonolactone", NUCLEOSIDES, NUCLEOTIDES & NUCLEIC ACIDS, vol. 29, no. 2, 26 February 2010 (2010-02-26), pages 113-122, XP55030987, ISSN: 1525-7770, DOI: 10.1080/15257771003597758
- YANA CEN ET AL.: "Diastereocontrolled electrophilic fluorinations of 2-deoxyribonolactone: syntheses of all corresponding 2-deoxy-2-fluorolactones and 2'-deoxy-2'-fluoro-NAD+s", JOURNAL OF ORGANIC CHEMISTRY., vol. 74, no. 16, 7 July 2009 (2009-07-07), pages 5779-5789, XP002757847, USAMERICAN CHEMICAL SOCIETY, WASHINGTON, DC. ISSN: 0022-3263
- TAKASHI NAKANO ET AL.: "Synthese von neuartigen difluorierten Prostaycclinen - Erhöhun der Stabilität durch Fluorsubstituenten", ANGEWANDTE CHEMIE, vol. 108, no. 9, 1996, pages 1082-1084, XP002757848, DEVCH VERLAG, WEINHEIM. ISSN: 0570-0833
- 'Nucleophilic substitution versus electron transfer. 1. The mechanism of electrophilic fluorinations' TETRAHEDRON LETTERS vol. 32, no. 31, 1991, pages 3815 - 3818, XP055196433
- 'Large-Scale Asymmetric Synthesis of the Bioprotective Agent JP4-039 and Analogs' ORGANIC LETTERS vol. 13, no. 9, 2011, pages 2318 - 2321, XP055196437

## Description

The present invention relates to a process for producing a difluoro lactone compound, which is characterized by selectively difluorinating the α-position of a carbonyl group without forming a hardly soluble by-product.

Difluoro ester compounds are important compounds as pharmaceuticals and agricultural chemicals, or as their intermediates. For example, intermediates for antineoplastic agents (L. W. Hertel et al., J. Org. Chem., 53, 2406 (1988)), intermediates for difluoro prostaglandins (JP-A-56-501319), difluoro peptides (S. Thaisrivongs et al., J. Med. Chem., 29, 2080 (1986)), etc. are known.

As electrophilic fluorinating agents to be used for preparing fluoro compounds, fluorine gas, xenon fluoride, perchloryl fluoride, etc. have been known since relatively long ago. Further, in recent years, electrophilic fluorinating agents such as N-fluoro sulfonimide, N-fluoro sulfonamide, etc. have also been used and are known, for example, by D.H.R. Barton et al. (USP 3,917,688, J. Chem. Soc. Perkin I, 732 (1974)), etc.

In fluorination by such electrophilic fluorinating agents, the fluorination is usually carried out by deprotonation at the α-position of an electron withdrawing group to prepare an active enolate in the system. However, such a reaction has some problems. Firstly, the substrate to be difluorinated is rather limited. For the difluorination to proceed, the substrate is limited to a compound which has electrophilic groups such as carbonyl groups, aromatic rings, sulfonyl groups, phosphoryl groups or carbon-carbon unsaturated bonds at both sides of the methylene group to be difluorinated, or a compound having an electrophilicity higher than a usual ketone, such as an aryl ketone, and in difluorination of a dialkyl ketone or ester, a mixture of a monofluoro product and a difluoro product is likely to be obtained. This is considered attributable to such that deprotonation by a base is more difficult in the case of the monofluoro product than the starting material, and the formed monofluoro enolate is unstable.

Secondly, in a case where a difluoro product and a monofluoro product are obtained as a mixture, the two products are similar in their physical and chemical properties such as their boiling points, polarity, etc., whereby it may sometimes be difficult to separate them by a separation method such as recrystallization, distillation or column chromatography.

In order to solve the above problems and to carry out difluorination of a compound with inadequate reactivity, a two-step difluorination reaction has been widely adopted wherein the monofluoro product is once isolated and then subjected to fluorination again. For example, by Yana Cen, et al. (J. Org. Chem., 5779 (2009)), deoxyribonolactone was reacted with N-fluorobenzene sulfonimide and lithium hexamethyldisilazide to obtain a monofluoro product, which was again reacted with the same reactants to obtain a difluoro product in a yield of 51%. However, this method cannot be regarded as a preferred method, since the number of steps increases as compared with the method of synthesizing the difluoro product directly.

In order to carry out the difluorination reaction in one step, a method of producing a difluoro compound selectively in a high yield has been proposed wherein a lactone or a carbonyl compound is reacted with N-fluorobenzene sulfonimide in the presence of a basic compound and a metal compound reactant such as manganese bromide or the like (Patent Documents 1 and 2). The desired difluoro compound is obtainable in a high yield, when a compound of a heavy metal such as manganese, zirconium or cerium is used as the metal compound reactant. However, in this method, a by-product which is derived from the heavy metal compound and which is hardly soluble in water or in an organic solvent, is formed, and there still remains a room for improvement in that separation between the desired product and the by-product, or the operation of cleaning the reaction container, tends to be cumbersome. Especially in the production of pharmaceuticals, contamination of even a very small amount of a heavy metal should not be permitted in many cases, and therefore, it is better not to use a heavy metal compound for the reaction. Further, there still remains a room for improvement also with respect to the reaction yield.
Patent Document 1: JP-A-8-143560
Patent Document 2: JP-A-9-110729
Patent Document 3: WO 2011/003018 A2 describes halogenated 2-deoxy-lactones, 2'-deoxy-nucleoside and derivatives thereof.
Patent Document 4: WO 2011/111714 A1 describes a synthesis of a novel EP4 agonist including a fluorination reaction in the presence of a metal compound.

Moreover, synthesis of new types of difluorinated prostacylines - improvement of stability by fluorine substitution is described by Nakano, T. et al. (Angew. Chem., 1996, 108, Nr. 9, 1082-1084).

It is an object of the present invention to provide a method for producing a difluoro lactone compound highly selectively in a high yield without forming a hardly soluble by-product.

The present invention provides the following constructions as its gist.
[1] A method for producing a difluoro lactone compound represented by the following formula (4), which comprises fluorinating a lactone compound represented by the following formula (3) by reacting it with an electrophilic fluorinating agent in the presence of potassium hexamethyldisilazide and in the absence of a metal compound reactant: 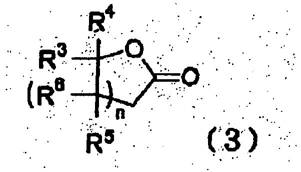 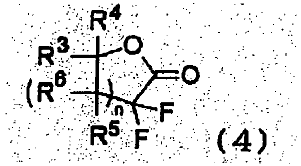 wherein each of R³, R⁴, R⁵ and R⁶ which are independent of one another, is a monovalent group selected from the group consisting of a hydrogen atom, a halogen atom, a protected hydroxy group, a protected amino group, a protected carboxy group and a C₁₋₂₀ hydrocarbon group which may have a substituent, or adjacent two among R³, R⁴, R⁵ and R⁶ are bonded to form a C₂₋₆ alkylene group which may have a substituent and other than the two among R³, R⁴, R⁵ and R⁶ are, each independently, the above monovalent group, and n is an integer of from 1 to 4.
[2] The method according to the above [1], wherein after conducting the fluorination reaction, a compound to decompose the remaining electrophilic fluorinating agent is added.
[3] The method according to the above [2], wherein the compound to decompose the electrophilic fluorinating agent is an amine or a halogen ion salt.
[4] The method according to any one of the above [1] to [3], wherein the electrophilic fluorinating agent is an electrophilic fluorinating agent selected from the group consisting of N-fluoro sulfonamides and N-fluoro sulfonimides.
[5] The method according to any one of the above [1] to [4], wherein the reaction is conducted at from -120°C to -50°C.
[6] The method according to any one of the above [1] to [5], wherein the ratio represented by the number of equivalent of the electrophilic fluorinating agent/the number of moles of the lactone compound represented by the formula (3) is from 1.6 to 12.
[7] The method according to any one of the above [1] to [6], wherein the ratio represented by the number of equivalent of potassium hexamethyldisilazide/the number of equivalent of the electrophilic fluorinating agent is from 0.5 to 2.0.
[8] The method according to any one of the above [1] to [7], wherein the lactone compound represented by the formula (3) is a lactone compound represented by the following formula (5): 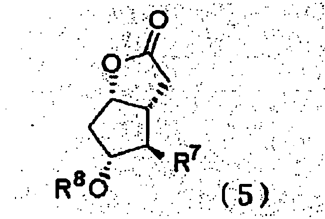 wherein R⁷ is a C₁₋₁₄ hydrocarbon group which may have a substituent, and R⁸ is a hydrogen atom or a protective group.
[9] The method according to any one of the above [1] to [7], wherein the lactone compound represented by the formula (3) is a compound represented by the following formula (9): 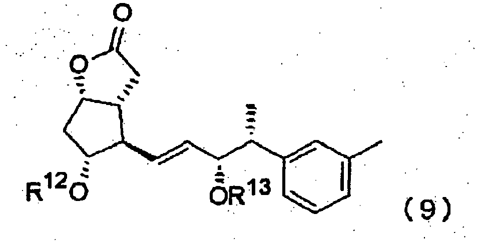 wherein each of R¹² and R¹³ which are independent of each other, is a tetrahydropyranyl group, a benzoyl group, a p-phenylbenzoyl group or a SiX₃ group, wherein X is an alkyl group, an aryl group, an aralkyl group or a heterocyclic group.
   Further, the present invention relates also to the following synthesis method using the difluoro lactone compound obtained by the above method.
[10] A method for producing a compound represented by the following formula (11), which comprises obtaining a difluoro lactone compound by the method as defined in the above [9], and further reacting the difluoro lactone compound with (4-(1 H-tetrazol-5-yl)butyl)triphenyl phosphonium bromide: 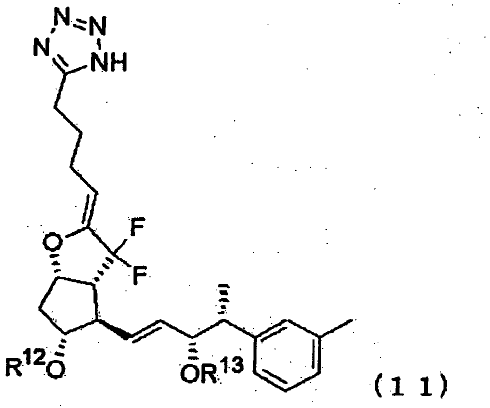 wherein each of R¹² and R¹³ which are independent of each other, is a tetrahydropyranyl group, a benzoyl group, a p-phenylbenzoyl group or a SiX₃ group, wherein X is an alkyl group, an aryl group, an aralkyl group or a heterocyclic group.
[11] A method for producing a compound represented by the following formula (12), which comprises obtaining a compound represented by the formula (11) by the method as defined in the above [10], and further eliminating R¹² and R¹³ of the compound represented by the formula (11) for substitution by hydrogen atoms.

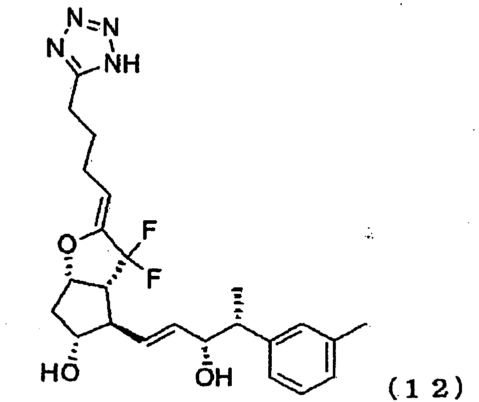

According to the production method of the present invention, it is possible to produce a difluoro lactone compound selectively in a high yield without using a metal compound reactant, whereby there is no trouble of inclusion of metal impurities, and there is no formation of a hardly soluble by-product.

In the following description, a "lower" organic group means a C₁₋₆ organic group and is preferably a C₁₋₄ organic group. An aralkyl group is an alkyl group having an aromatic ring bonded at its terminal. An alkoxime group is a compound having OH of an oxime substituted by OC.

From the lactone represented by the formula (3), a difluoro lactone represented by the formula (4) will be obtained. 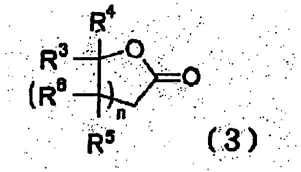 (wherein each of R³, R⁴, R⁵ and R⁶ which are independent of one another, is a monovalent group selected from the group consisting of a hydrogen atom, a halogen atom, a protected hydroxy group, a protected amino group, a protected carboxy group and a C₁₋₂₀ hydrocarbon group which may have a substituent, or adjacent two among R³, R⁴, R⁵ and R⁶ are bonded to form a C₂₋₆ alkylene group which may have a substituent and other than the two among R³, R⁴, R⁵ and R⁶ are, each independently, the above monovalent group, and n is an integer of from 1 to 4.). 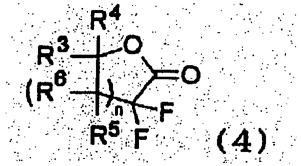 (wherein each of R³, R⁴, R⁵ and R⁶ are the same as in the above formula (3).).

As the protected hydroxy group in R³, R⁴, R⁵ and R⁶ in the formula (3), a hydroxy group protected by a known or well known protective group to be used as a protective group for a hydroxy group may be employed. As such a protective group, for example, a triorganosilyl group represented by the formula SiX₃ (X is an alkyl group, an aryl group, an aralkyl group and a heterocyclic group), an acyl group, a cyclic ether group, a C₁₋₂₀ alkyl group which may have a substituent and an aralkyl group may be used. As the triorganosilyl group, a triorganosilyl group having 3 groups selected from lower alkyl groups and aryl groups, is preferred. Specifically, a t-butyldimethylsilyl group, a t-butyldiphenylsilyl group, a triethylsilyl group, a triphenylsilyl group and a triisopropylsilyl group are preferred. As the acyl group, an acetyl group, a benzoyl group or a p-phenylbenzoyl group is, for example, preferred. As the cyclic ether group, a tetrahydropyranyl group or a tetrahydrofuranyl group is, for example, preferred. Further, as the alkyl group which may have a substitutent, an alkoxyalkyl group such as a methoxymethyl group, a 1-ethoxyethyl group or a 2-methoxyethoxymethyl group is, for example, preferred. As the aralkyl group, a benzyl group, a methoxybenzyl group or a trityl group is, for example, preferred.

As the protected amino group in R³, R⁴, R⁵ and R⁶ in the formula (3), an amino group protected by a known or well known protective group to be used as a protective group for an amino group may be employed. As such a protective group, for example, an acyl group, an alkoxycarbonyl group, an alkyl group, an alkenyl group, an aralkyl group, a triorganosilyl group and a sulfonyl group may be mentioned. As the acyl group, an acetyl group, a benzoyl group or a trifluoroacetyl group is, for example, preferred. As the alkoxycarbonyl group, a t-butoxycarbonyl group or a benzyloxycarbonyl group is, for example, preferred. As the alkyl group, the alkenyl group and the alkynyl group, a methoxymethyl group, an allyl group, a benzyl group, a trityl group and a methoxybenzyl group are preferred. As the triorganosilyl group, a t-butyldimethylsilyl group, a t-butyldiphenylsilyl group, a triethylsilyl group, a triphenylsilyl group or a triisopropylsilyl group is, for example, preferred. As the sulfonyl group, a p-toluenesulfonyl group, a benzenesulfonyl group, a p-chlorobenzenesulfonyl group, a p-nitrobenzenesulfonyl group or a methanesulfonyl group is, for example, preferred.

As the protected carboxy group in R³, R⁴, R⁵ and R⁶ in the formula (3), a carboxy group protected by a known or well known protective group to be used as a protective group for a carboxy group or its synthon may be employed. As such a protective group, for example, an alkyl group, an alkenyl group, an aralkyl group, a triorganosilyl group or an ortho ester is preferred. As the alkyl group, the alkenyl group and the aralkyl group, a methoxymethyl group, an allyl group, a benzyl group, a trityl group and a methoxybenzyl group are preferred. As the triorganosilyl group, a t-butyldimethylsilyl group, a t-butyldiphenylsilyl group, a triethylsilyl group, a triphenylsilyl group or a triisopropylsilyl group is, for example, preferred.

As the synthon, a tetrazole group is, for example, preferred.

The protective group in the protected hydroxy group, the protected amino group or the protected carboxy group as described above, can be eliminated by a usual method. For example, such a protected group can be converted to a hydroxy group, an amino group or a carboxy group easily by a method disclosed in literatures such as "Shin Jikken Kagaku Koza (New Experimental Chemistry Handbook) 14, Syntheses and Reactions (I), (II) and (V) of Organic Compounds" (Maruzen Publishing Co., Ltd), "Protective Groups in Organic Syntheses" (edited by T.W. Greene, J. Wiley & Sons).

The hydrocarbon group in R³, R⁴, R⁵ and R⁶ in the formula (3) may be linear, branched or cyclic and is preferably a C₁₋₂₀ alkyl group, a C₃₋₂₀ cycloalkyl group, a C₂₋₂₀ alkenyl group, a C₃₋₂₀ cycloalkenyl group, a C₂₋₂₀ alkynyl group, a C₃₋₂₀ cycloalkynyl group or a C₆₋₂₂ aryl group.

In the formula (3), n is an integer of from 1 to 4. That is, the compound represented by the formula (3) is a 5- to 8-membered ring lactone. n is preferably 1 or 2. That is, the compound represented by the formula (3) is preferably a 5- or 6-membered ring lactone. Such a lactone may be such that two among R³, R⁴, R⁵ and R⁶ are bonded to form a cycloalkylene group.

As the lactone represented by the formula (3), a lactone represented by the following formula (5) is more preferred. The lactone represented by this formula (5) is such a compound that in the formula (3), n is 1, each of R⁴ and R⁵ is a hydrogen atom, and R⁶ and R³ are bonded to form a trimethylene group, and substituents R⁷ and OR⁸ are bonded to such a trimethylene group, and further, the compound has a specific structure shown by the formula (5). The lactone represented by this formula (5) has the same skeleton as a partial structure of a prostaglandin I2 (hereinafter PGI2) and is a known compound as an intermediate for the synthesis of PGI2 [a derivative of so-called Corey lactone]. 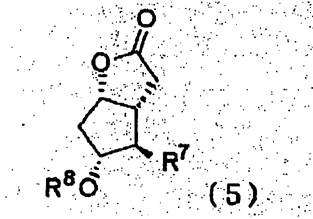 (wherein R⁷ is a C₁₋₁₄ hydrocarbon group which may have a substituent, and R⁸ is a hydrogen atom or a protective group.).

The hydrocarbon group in R⁷ may be linear, branched or cyclic and is preferably a C₁₋₁₄ alkyl group, a C₃₋₁₄ cycloalkyl group, a C₂₋₁₄ alkenyl group, a C₃₋₁₄ cycloalkenyl group, a C₂₋₁₄ alkynyl group, a C₃₋₁₄ cycloalkynyl group or a C₆₋₁₀ aryl group.

In a case where R⁸ is a protective group, the protective group is a protective group for a hydroxy group, and its embodiments and preferred embodiments are the same as for the protective group in the protected hydroxy group in R³, R⁴, R⁵ and R⁶ in the above formula (3).

A difluorolactone of the formula (6) obtainable by the method of the present invention from the lactone represented by the formula (5) is useful as an intermediate for a difluoroprostaglandin. 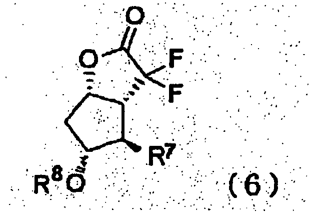 (wherein R⁷ and R⁸ are as defined above.).

R⁷ in the formula (5) or (6) is preferably a group corresponding to a ω-chain portion of natural PGI2, a group corresponding to a ω-chain portion of various PGI2, or a group which can readily be converted to such a ω-chain portion. It is particularly preferred that at least one type of the substituent in R⁷ is the protected hydroxy group. More preferred R⁷ is a group represented by the following formula (7) or (8).

-A-CH(OR¹⁰)-R⁹ (7)

-CH₂OR¹¹ (8)

In the formula (7), A is a vinylene group, an ethynylene group or an ethylene group, preferably a vinylene group or an ethynylene group, most preferably a vinylene group which is the same as one corresponding to A in natural PGI2.

R⁹ is preferably a group corresponding to a ω-chain portion of natural PGI2 or a group corresponding to a ω-chain portion of various PGI2. As such a group, a C₁₋₁₀ hydrocarbon group which may have a substituent is preferred. Such a hydrocarbon group may be linear, branched or cyclic and may, for example, be a C₁₋₁₀ alkyl group, a C₃₋₁₀ cycloalkyl group, a C₁₋₁₀ alkenyl group, a C₃₋₁₀ cycloalkenyl group, a C₁₋₁₀ alkynyl group, a C₈₋₁₂ cycloalkynyl group or a C₆₋₁₀ aryl group.

R⁹ is preferably a chain hydrocarbon group, particularly preferably a C₃₋₈ alkyl group which may have a substituent, a C₃₋₈ alkenyl group which may have a substituent or a C₃₋₈ alkynyl group which may have a substituent. Such a C₅₋₆ linear group which may have a substituent, or its mono-methyl or di-methyl substitute, is more preferred. Such a group may specifically be a n-propyl group, a n-pentyl group, a n-octyl group, a 2-methylhexyl group, a 1-methyl-3-pentenyl group, a 1-methyl-3-hexynyl group, a 1,1-dimethyl-3-pentynyl group or a 1,1-dimthyl-3-hexynyl group. Among them, a n-pentyl group, a 2-methylhexyl group, a 1-methyl-3-pentyl group, a 1-methyl-3-hexynyl group, or a 1,1-dimethyl-3-hexynyl group, is preferred.

Each of R¹⁰ and R¹¹ is a hydrogen atom or a protective group (protective group for a hydroxy group).

In a case where each of R⁸, R¹⁰ and R¹¹ is a protective group (protective group for a hydroxy group), the protective group is not particularly limited, and the same protective group as the protective group for the protected hydroxy group in R³, R⁴, R⁵ and R⁶ in the above formula (3) may be employed. Such protective groups may be the same or different from one another. Such protective groups are adopted depending upon the particular purpose. For example, in a case where it is required to selectively deprotect only one protective group of a compound having two protective groups, it is preferred to employ protective groups which are different in the reactivity. Specifically, in a case where a triorganosilyl group or a cyclic ether group is used as R⁸ or R¹⁰, it is preferred to employ, as R¹¹, a protective group which is the same or different from R⁸ or R¹⁰, and which has a reactivity different from R⁸ or R¹⁰.

In a case where each of the above R³, R⁴, R⁵, R⁶, R⁷ and R⁹ is a group which may have a substituent, the substituent is not particularly limited. The substituent may, for example, be a hydrocarbon group such as an alkyl group, a cycloalkyl group, an alkenyl group, a cycloalkenyl group, an alkynyl group, a cycloalkynyl group or an aryl group; a halogen atom such as a fluorine atom, a chlorine atom, a bromine atom or an iodine atom; an oxygen-containing group such as an oxo group, an alkoxy group, a hydroxy group, a protected hydroxy group, a carbonyl group, a carboxy group, a carboxy salt group or a protected carboxy group; a nitrogen-containing group such as an amino group, a protected amino group, a nitro group, a cyano group, a carbamoyl group, a urethane group, an isocyano group or an alkoxime group; a sulfur-containing group such as a thioformyl group, a dithiocarboxy group, a sulfonyl group, an alkylsulfonyl group, an arylsulfonyl group, an alkylsulfinyl group, an arylsulfinyl group, an alkylsulfenyl group or an arylsulfenyl group; a phosphorus-containing group such as a phosphoryl group or its salt; or a heterocyclic group such as piridyl group, an imidazolyl group, an indolyl group, a quinolyl group, a furyl group or a thienyl group. The hydrocarbon group may be linear, branched or cyclic. Further, the substituent may be a group having the above groups combined, such as a hydrocarbon group having its hydrogen atoms substituted by halogen atoms.

Further, the protected hydroxy group, the protected carboxy group and the protected amino groups may be those mentioned above.

The production method of the present invention is conducted in the absence of a metal compound reactant. In the present invention, the metal compound reactant means a metal compound reactant disclosed in Patent Documents 1 and 2. More specifically, a metal compound containing a metal species selected from the group consisting of B, Mg, Al, Ca, Ti, V, Mn, Fe, Co, Ni, Cu, Zn, Zr, Sn, Ba, Hf, W, La, Ce and Sm may be mentioned. As the metal compound containing such a metal species, an organic metal compound or a metal salt may, for example, be mentioned.

The electrophilic fluorinating agent to be used in the production method of the present invention is not particularly limited, and a known or well known electrophilic fluorinating agent may be employed. For example, it is possible to use an electrophilic fluorinating agent disclosed in a literature such as "Fusso no Kagaku (Chemistry of fluorine)" edited by Tomoya Kitazume, Takashi Ishihara and Takeo Taguchi (Kodansha Scientific). Specifically, an N-fluoro sulfonamide or an N-fluoro sulfonimide is preferred. More specifically, N-fluorobenzenesulfonimide, N-fluoro-p-fluorobenzenesulfonimide, N-fluoro-o-benzenedisulfonimide, N-fluoro-p-toluenesulfonimide, N-fluoro-N-t-butylbenzenesulfonamide, N-fluoro-N-t-butyl-p-toluenesulfonamide, N-fluoro-N-methylbenzenesulfonamide or N-fluoro-N-norbornyl-p-fluorobenzenesulfonamide is preferred, and N-fluorobenzenesulfonimide is more preferred.

The amount of the electrophilic fluorinating agent is not particularly limited, and it is preferred to use at least an amount capable of giving fluorine atoms required for the desired difluorination. That is, the ratio represented by the number of equivalent of the electrophilic fluorinating agent/the number of moles of the lactone compound represented by the above formula (3) is preferably from 1.6 to 12, more preferably from 2.0 to 6.0, further preferably from 2.0 to 5.0, most preferably from 3.0 to 5.0.

Here, the number of equivalent of the electrophilic fluorinating agent means the number of fluorine atoms which can be supplied by one molecule of the electrophilic fluorinating agent x the number of moles of the electrophilic fluorinating agent.

The basic compound to be used in the production method of the present invention is a basic compound which is not the above metal compound reactant and which is not a metal compound containing the above metal species.

As such a basic compound, potassium hexamethyldisilazide is used.

With respect to the amount of the basic compound to be used for the production method of the present invention, since the basic compound and the electrophilic fluorinating agent may sometimes react with each other, it is preferred that one of them should not be excessive as compared to the other. From such a viewpoint, the ratio represented by the number of equivalent of the basic compound/the number of equivalent of the electrophilic fluorinating agent is preferably from 0.5 to 2.0, more preferably from 0.5 to 1.5. Here, the number of equivalent of the basic compound means the valency of the basic compound x the number of moles of the basic compound. The meaning of the number of equivalent of the electrophilic fluorinating agent is as mentioned above. In a case where the lactone compound as the starting material, or the product, is likely to be decomposed by the basic compound, the above ratio of the number of equivalent of the basic compound/the number of equivalent of the electrophilic fluorinating agent is preferably at most 1.0. Specifically, the ratio represented by the number of equivalent of the basic compound/the number of equivalent of the electrophilic fluorinating agent is preferably from 0.5 to 1.0, more preferably from 0.8 to 1.0.

Here, in a case where the lactone compound as the starting material has a group reactive with the basic compound, such as a hydroxy group, an excess amount of the basic material to be consumed by the reaction with such a group is required. For example, the case of using, as the starting material, a compound represented by the above-mentioned formula (5) wherein R⁸ is hydrogen, corresponds to such a case. In such a case, in addition to the basic compound in an amount corresponding to the above-mentioned ratio represented by the number of equivalent of the basic compound/the number of equivalent of the electrophilic fluorinating agent, it is required to excessively use the basic compound in an amount to be consumed by the reaction with the group reactive with the basic compound.

The production method of the present invention is carried out in the presence of a solvent, and as such a solvent, an inert solvent is preferred. The inert solvent is a solvent which is unreactive with the basic compound or the electrophilic fluorinating agent at the reaction temperature. As such an inert solvent, an ether type solvent, a hydrocarbon type solvent, a polar solvent or a mixed solvent thereof is preferred. As the ether type solvent, preferred are diethyl ether, tetrahydrofuran, 1,4-dioxane, dimethoxyethane, diglyme and t-butyl methyl ether; as the hydrocarbon type solvent, preferred are hexane, toluene, benzene, pentane, xylene and petroleum ether; and as the polar solvent, preferred are dimethyl sulfoxide, hexamethylphosphoramide (HMPA), 1,3-dimethyl-3,4,5,6-tetrahydro-2(1H)-pirimidinone (DMPU), 1,3-dimethyl-2-imidazolidinone (DMI) and N,N,N',N'-tetramethylethylenediamine (TMEDA). In a usual case, the amount of the solvent is preferably from 5 to 1,000 parts by weight, more preferably from 10 to 100 parts by weight, per 1 part by weight of the compound represented by the formula (3).

In the production method of the present invention, the reaction temperature is preferably from -150 to 0°C, more preferably from -120 to 0°C, further preferably from-120 to -50°C, most preferably from -115 to -70°C. Usually, the lower the reaction temperature, the higher the selectivity for the desired fluorination reaction. Therefore, by carrying out the reaction at a temperature as low as possible within a range where the fluorination proceeds at a practically sufficient speed, it is possible to obtain the difluoro product in a high yield while preventing formation of a monofluoro by-product.

In the production method of the present invention, the order of addition of each compound and the electrophilic fluorinating agent may be such that the lactone compound and the electrophilic fluorinating agent be mixed, and then the basic compound be added, or the lactone compound and the basic compound be mixed, and then the electrophilic fluorinating agent be added. In a case where the lactone compound is likely to be decomposed by a basic material, it is preferred to employ a method wherein the lactone compound and the electrophilic fluorinating agent are preliminarily dissolved and mixed in a solvent, and when the temperature has reached a predetermined reaction temperature, the basic compound is added. By adopting such an addition order, it is considered that an excessive basic compound not used for the reaction with the lactone compound will react with and be consumed by the electrophilic fluorinating agent, whereby it is possible to prevent decomposition of the lactone compound or the product by the basic compound.

The reaction time in the production method of the present invention is preferably from 5 minutes to 24 hours at the predetermined reaction temperature, although it may depend on e.g. the reactivity of the lactone compound. Further, thereafter, it is preferred to raise the temperature to a predetermined temperature to stop the reaction in from 1 to 72 hours.

As a post treatment method for this reaction, it is possible to employ a method commonly known in an organic synthesis. For example, the reaction can be terminated by adding a compound (hereinafter referred to as a quenching agent) capable of supplying protons, such as water, an aqueous solution or an alcohol, in a large excess amount to the base used for the reaction. The temperature of the quenching agent and the reaction solution at the time of adding such a quenching agent may be in a range where the solvent used will not be solidified or boiled. In a case where the product is likely to be decomposed at a high temperature, the temperature of the reaction solution at the time of adding the quenching agent is preferably at most 40°C, more preferably at most 25°C, further preferably at most 0°C. Further, the addition of the quenching agent may be made at a low temperature in a range where the quenching agent or the solvent will not be solidified.

After termination of the reaction, extraction by liquid-liquid separation is carried out by adding an organic solvent and, as the case requires, water or an aqueous solution for adjustment to a proper acidity, and the organic phase is concentrated to recover the desired compound. The organic solvent to be used for the extraction by liquid-liquid separation is not particularly limited, and for example, it is possible to use hexane, ethyl acetate, diethyl ether, t-butyl methyl ether, chloroform or methylene chloride.

In this reaction, even after the reaction, the electrophilic fluorinating agent may frequently remain in the reaction system, whereby the difluorinated desired product and the electrophilic fluorinating agent may react during the post treatment operation to cause deterioration of the yield of the desired product. The electrophilic fluorinating agent is active also as an oxidizing agent, and therefore, in a case where the desired product has, in its molecule, a group reactive with the electrophilic fluorinating agent or the oxidizing agent, the deterioration of the yield is likely to be distinct. A functional group reactive with the electrophilic fluorinating agent or the oxidizing agent, may, for example, be an alkene, an alkyne, an alcoholic hydroxy group, an allyl ether, an allyl alcohol, an aldehyde, an acetal, a silyl ether, a thiol, a sulfide, a sulfoxide or an amino group. Among them, a particularly reactive functional group may be an alkene, an allyl ether, an allyl alcohol or a silyl ether.

In such a case that the electrophilic fluorinating agent remaining in the reaction solution presents an adverse effect in the post treatment step, a compound (hereinafter referred to as "a decomposing agent") to decompose the electrophilic fluorinating agent may be added. Such a decomposing agent may be added before adding the quenching agent or thereafter, but preferably before. The decomposing agent may be one having a reactivity such as nucleophilicity or reducing character to the electrophilic fluorinating agent. As such a decomposing agent, it is possible to employ ammonia, an amine, a hydroxide ion, an alkoxide, a salt of a halogen ion, etc. Ammonia may be added in a state of a gas, an aqueous solution or a solution in another solvent.

As the amine, any one of a primary amine, a secondary amine and a tertiary amine may be used, and for example, methylamine, hydroxylamine, diethylamine, morpholine, piperidine, 2-methoxyethylamine, 3-quinuclidinol or triethylamine may be mentioned. The amine is particularly preferably a C₁₋₁₈ trialkylamine, more preferably a C₁₋₈ trialkylamine, wherein the three alkyl groups are independent of one another.

The hydroxide ion may, for example, be sodium hydroxide or potassium hydroxide. The alkoxide may, for example, be sodium methoxide, sodium ethoxide or potassium t-butoxide. The salt of a halogen ion may, for example, be an iodide salt, a bromide salt or a chloride salt, preferably an iodide salt or a bromide salt, more preferably an iodide salt. The iodide salt may, for example, be ammonium iodide or potassium iodide. The bromide salt may, for example, be potassium bromide.

Among them, an amine or a salt of a halogen ion is particularly preferred, and at least one member selected from the group consisting of triethylamine and an iodide salt is more preferred, in that the reactivity with the electrophilic fluorinating agent, particularly with an N-fluorosulfonamide or an N-fluorosulfonimide, is particularly high. By using such a decomposing agent, it is possible to let only the decomposition reaction of the electrophilic fluorinating agent proceed selectively, while preventing a reaction of the desired product and the post treatment agent such as the extraction solvent. The addition temperature of the decomposing agent is preferably from -50 to 40°C, particularly preferably from -30 to 25°C, most preferably from -20 to 0°C. By adjusting the temperature within such a range, it is possible to prevent decomposition of the desired product, while increasing the reaction rate of the decomposing agent and the electrophilic fluorinating agent.

The compound represented by the formula (4) obtainable by the reaction is an important intermediate which can be led to pharmaceuticals containing various difluoro units. For example, a compound 10 represented by the following formula (10) obtainable from a compound 9 represented by the following formula (9) by the production method of the present invention, can be led, via a compound 11 represented by the following formula (11) and further by elimination of R¹² and R¹³ to form hydroxy groups, to a compound 12 represented by the following formula (12): 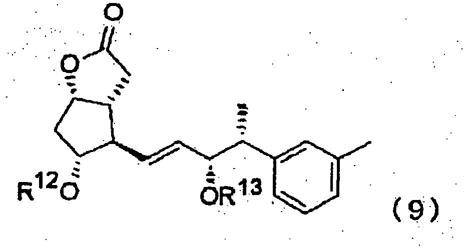 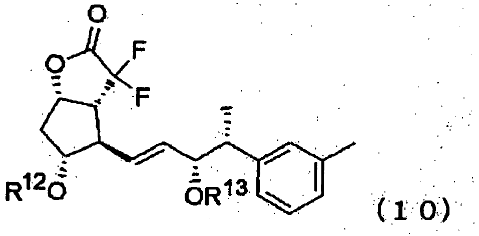 (wherein each of R¹² and R¹³ which are independent of each other, is a tetrahydropyranyl group, a benzoyl group, a p-phenylbenzoyl group or a SiX₃ group (X is an alkyl group, an aryl group, an aralkyl group or a heterocyclic group)), 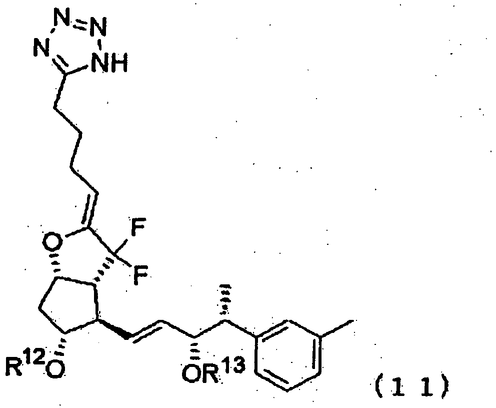 (wherein R¹² and R¹³ are as defined above), 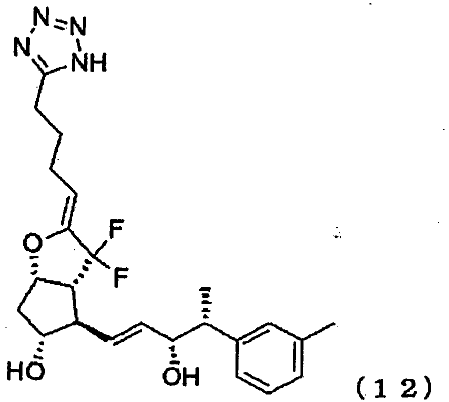 The compound 12 is useful as an EP4 agonist. Such an EP4 agonist is disclosed in WO2011 /111714.

### EXAMPLES

Now, the present invention will be described with reference to Examples. However, it should be understood that the present invention is by no means restricted by these Examples. NMR used in the following was JNM-AL300, manufactured by JEOL Ltd.

### [Example 1]

### Synthesis of (3aR,4R,5R,6aS)-5-((t-butyldimethylsilyl)oxy)-4-((3R,4R,E)-3-((t-butyldimethylsilyl)oxy)-4-(m-tolyl)pent-1-en-1-yl)-3,3-difluorohexahydro-2H-cyclopenta[b]furan-2-one (compound 10)

A solution of 1.0 g (1.84 mmol) of (3aR,4R,5R,6aS)-5-((t-butyldimethylsilyl)oxy)-4-((3R,4R,E)-3-((t-butyldimethylsilyl)oxy)-4-(m-tolyl)pent-1-en-1-yl)hexahydro-2H-cyclopenta[b]furan-2-one (compound 9), 2.3 g (7.34 mmol, 7.34 meq) of N-fluorobenzenesulfonimide (NFSI), 44 ml of THF and 13 ml of toluene, was cooled to - 100°C, and 6.4 ml (6.4 mmol, 6.4 meq) of a 1M THF solution of potassium hexamethyldisilazide was added. The reaction solution was stirred at -100°C for 30 minutes, then the temperature was raised to 0°C over a period of 1 hour, then 2.0 ml of triethylamine was added and stirred, and 50 ml of water was added for liquid-liquid separation, whereupon the aqueous phase was extracted with 30 ml of hexane. The organic phase was concentrated, and then the crude product was analyzed by NMR, whereby no N-fluorobenzenesulfonimide was detected. The residue deposited on the reaction container was all dissolved and removed by washing with methanol and water. The crude product was purified by silica gel flash chromatography using hexane and ethyl acetate as developing solvents to obtain 0.91 g (yield: 85%) of compound 10. The structural characteristics of the obtained compound 10 are as follows.
1H-NMR (CDCl₃, units for δ-values are all ppm, the same applies in the following Examples): δ-0.08-0.03 (m, 12H), 0.82 (s, 9H), 0.89 (s, 9H), 1.28 (d, J=7.0Hz, 3H), 1.70-1.77 (m, 1H), 1.96-2.04 (m, 1H), 2.31 (s, 3H), 2.60-2.91 (m, 3H), 3.82-3.87 (m, 1H), 3.99-4.23 (m, 1H), 5.00 (t, J=6.4Hz, 1H), 5.06 (dd, J=15.7, 7.8Hz, 1H), 5.33 (ddd, J=15.9, 6.7, 1.2Hz, 1H), 6.88-7.16 (m, 4H).
19F-NMR (CDCl3): -113.1 (d, J=279.3Hz), -91.0 (dd, J=279.3, 25.9Hz)

### [Examples 2 to 13]

The reaction was carried out under the same conditions as in Example 1 except that the reaction temperature, the molar ratio of the equivalent of NFSI/compound 9, the quench condition and the ratio of the equivalent of the basic compound/the number of moles of compound 9 were changed as shown in Table 1. Here, the details of the quench condition are as follows.
Quench condition A: Quenching at 0°C with 5% sodium bicarbonate water, was followed by extraction with an organic solvent (hexane/ethyl acetate = 1:1).
Quench condition B: A 5% ammonium iodide aqueous solution was added at 0°C, followed by stirring at room temperature for 5 minutes, and then, a 10% sodium thiosulfate aqueous solution was added, followed by extraction with an organic solvent (hexane/ethyl acetate = 1:1, or hexane).
Quench condition C: Triethylamine in a molar amount twice of NFSI was added at 0°C, followed by stirring at 0°C for 5 minutes, and then water was added at room temperature, followed by extraction with an organic solvent (hexane).

### [Comparative Example 1]

To 1.48 g of manganese bromide and 2.48 g of N-fluorobenzenesulfonimide, 19 mL of tetrahydrofuran (THF) was added, followed by stirring for 30 minutes and then by cooling to -78°C. A THF (5 mL) solution containing 0.5 g of compound 9 was added, and then, a toluene solution (0.5 M, 13 mL) of potassium bis(trimethylsilyl)amide was added, followed by stirring for 30 minutes, and then, the temperature was raised to 0°C over a period of 3 hours. The reaction solution was poured into saturated sodium bicarbonate water, followed by extraction with hexane/ethyl acetate = 1:1 mixture. The extract was dried over magnesium sulfate and then concentrated under reduced pressure. The crude product was analyzed by NMR, whereby non-reacted N-fluorobenzenesulfonimide was detected. A residue derived from manganese bromide was deposited on the reaction container, and it was not removed by washing with an organic solvent or water. It was necessary to carry out washing by means of fuming nitric acid. The crude product was purified by silica gel column chromatography (hexane/ethyl acetate = 20:1) to obtain 0.32 g (yield: 60%) of compound 10.

### [Comparative Example 2]

The reaction was carried out under the same conditions as in Comparative Example 1 except that the reaction temperature, the amount of manganese bromide, the molar ratio of NFSI to compound 9, the quench condition and the ratio of the basic compound to compound 9 were changed as shown in Table 1.

**[Table 1]**

| | Temperature (°C) | Number of moles of metal compound/ number of moles of lactone compound | Number of equivalent of NFSI/number of moles of lactone compound | Number of equivalent of basic compound/ number of equivalent of NFSI | Quench condition | Yield (%) of difluoro product | Insoluble residue | Residual NFSI |
|---|---|---|---|---|---|---|---|---|
| Ex. 1 | -100 | Nil | 4 | 0.87 | C | 85 | Nil | Nil |
| Ex. 2 | -100 | Nil | 4 | 0.88 | B | 83 | Nil | Nil |
| Ex. 3 | -100 | Nil | 4 | 0.88 | A | 79 | Nil | Present |
| Ex. 4 | -100 | Nil | 8.8 | 0.91 | B | 50 | Nil | Nil |
| Ex. 5 | -100 | Nil | 6 | 0.88 | B | 72 | Nil | Nil |
| Ex. 6 | -100 | Nil | 3 | 0.90 | B | 58 | Nil | Nil |
| Ex. 7 | -100 | Nil | 2.5 | 0.88 | B | 45 | Nil | Nil |
| Ex. 8 | -115 | Nil | 4 | 0.88 | C | 86 | Nil | Nil |
| Ex. 9 | -84 | Nil | 4 | 0.88 | B | 68 | Nil | Nil |
| Ex. 10 | -78 | Nil | 6 | 0.88 | B | 58 | Nil | Nil |
| Ex. 11 | -78 | Nil | 4 | 0.88 | B | 68 | Nil | Nil |
| Ex. 12 | -45 | Nil | 4 | 0.75 | A | 25 | Nil | Present |
| Ex. 13 | 0 | Nil | 4 | 0.75 | A | 20 | Nil | Present |
| Comp. Ex. 1 | -78 | 7.5 | 8.6 | 0.83 | A | 60 | Present | Present |
| Comp. Ex. 2 | -100 | 6 | 6 | 0.88 | B | 43 | Present | Nil |

### [Example 14]

### Synthesis of 4-[(Z)-(1S,5R,6R,7R)-6-[(1E,3R,4R)-3-t-butyldimethylsiloxy-4-(m-tolyl)-1-pentenyl]-7-t-butyldimethylsiloxy-2-oxa-4,4-difluoro-bicyclo[3.3.0]octan-3-ylidene]-1-(tetrazol-5-yl)butane (compound 11)

To a suspension of 0.81 kg of (4-(1H-tetrazol-5-yl)butyl)triphenyl phosphonium bromide in 12.6 L of toluene, 3.5 L of a 1M THF solution of potassium hexamethyldisilazide was added at room temperature, followed by stirring at 60°C for 1 hour. After cooling the liquid to -15°C, the solution of 0.25 kg of (3aR,4R,5R,6aS)-5-((t-butyldimethylsilyl)oxy)-4-((3R,4R,E)-3-((t-butyldimethylsilyl)oxy)-4-(m-tolyl)pent-1-en-1-yl)-3,3-difluorohexahydro-2H-cyclopenta[b]furan-2-one (compound 10) obtained in Example 1 in 5.0 L of toluene was added, followed by stirring at -15°C for 30 minutes and then at 0°C for 20 hours. To the reaction solution, 15.6 L of a 4% sodium dihydrogen citrate aqueous solution was added, followed by liquid-liquid separation. The aqueous phase was extracted with 12.6 L of a mixed liquid of hexane:ethyl acetate = 5:1. The organic phase was concentrated and then purified by silica gel flash chromatography using hexane and ethyl acetate as developing solvents to obtain 0.28 kg (yield: 95%) of compound 11. The structural characteristics of compound 11 are as follows.
1H-NMR (CDCl3): δ-0.14-0.01 (m, 12H), 0.82 (s, 9H), 0.89 (s, 9H), 1.23-1.27 (m, 3H), 1.82-2.09 (m, 5H), 2.21-2.28 (m, 1H), 2.31 (s, 3H), 2.45-2.53 (m, 1H), 2.64-2.73 (m, 2H), 2.93-2.97 (m, 2H), 3.90 (dd, J=11.7, 5.3Hz, 1H), 4.08-4.09 (m, 1H), 4.84-4.87 (m, 2H), 5.27 (dd, J=15.5, 7.8Hz, 1H), 5.44 (dd, J=15.6, 6.2Hz, 1H), 6.92-7.16 (m, 4H).
19F-NMR (CDCl3): -112.3 (d, J=253.4Hz), -81.4 (dd, J=253.4, 18.7Hz)

### [Example 15]

### Synthesis of 4-[(Z)-(1S,5R,6R,7R)-6-[(1 E,3R,4R)-3-hydroxy-4-(m-tolyl)-1-pentenyl]-7-hydroxy-2-oxa-4,4-difluoro-bicyclo[3.3.0]octan-3-ylidene]-1-(tetrazol-5-yl)butane (compound 12)

To a suspension having 1.5 g (2.2 mmol) of 4-[(Z)-(1S,5R,6R,7R)-6-[(1E,3R,4R)-3-t-butyldimethylsiloxy-4-(m-tolyl)-1-pentenyl]-7-t-butyldimethylsiloxy-2-oxa-4,4-difluoro-bicyclo[3.3.0]octan-3-ylidene]-1-(tetrazol-5-yl)butane (compound 11) obtained in Example 14, 27 ml of acetonitrile and 3 ml of water put together, 0.60 g (4.4 mmol) of sodium hydrogen sulfate monohydrate was added, followed by stirring in air at room temperature. Upon expiration of 24 hours, the liquid was uniform, and after confirming disappearance of the raw material by thin-layer chromatography, 60 ml of 1.2% sodium bicarbonate water was added, followed by washing three times with 27 ml of heptane. To the acetonitrile/water mixed liquid phase, 1.2 g of sodium hydrogen sulfate was added, followed by extraction with 27 ml of ethyl acetate, and the organic phase was washed with 30 ml of a 5% sodium chloride aqueous solution. The organic phase was concentrated under reduced pressure to obtain 1.1 g of a solid, which was analyzed by NMR and HPLC, whereby the yield of 4-[(Z)-(1S,5R,6R,7R)-6-[(1E,3R,4R)-3-hydroxy-4-(m-tolyl)-1-pentenyl]-7-hydroxy-2-oxa-4,4-difluoro-bicyclo[3.3.0]octan-3-ylidene]-1-(tetrazol-5-yl)butane (compound 12) was 98%. The structural characteristics of compound 12 are as follows.
1H-NMR (CD3OD): δ 1.30 (d, J=7.0 Hz, 3H), 1.69 (dddd, J=14.6, 7.6, 3.0, 2.6 Hz, 1H), 1.82-1.95 (m, 2H), 2.10-2.16 (m, 2H), 2.29 (s, 3H), 2.31-2.41 (m, 2H), 2.48-2.56 (m, 1H), 2.72 (q, J=7.0 Hz, 1H), 2.93 (t, J=7.6 Hz, 2H), 3.78 (q, J=7.6 Hz, 1H), 4.04-4.10 (m, 1H), 4.69 (dt, J=6.48, 2.96 Hz, 1H), 4.79 (dt, J=7.6, 5.0 Hz, 1H), 5.36-5.46 (m, 2H), 6.95-7.13 (m, 4H).
19F-NMR (CD3OD): -116.6 (d, J=250.5 Hz), -84.8 (ddd, J=251.9, 17.3, 14.4 Hz)

### [Example 16]

A solution of 0.50 g of 2-naphthyl dodecanoate, 1.95 g of N-fluorobenzenesulfonimide, 22 ml of THF and 6.5 ml of toluene, was cooled to -78°C, and 5.36 ml of a 1.0M THF solution of potassium hexamethyldisilazide was added. After raising the temperature to room temperature, an aqueous citric acid solution was added to terminate the reaction, followed by extraction with ethyl acetate, and the solvent was removed. 1.09 g of the obtained product was quantitatively analyzed by 19F NMR, whereby it was confirmed that 2-naphthyl 2-fluorododecanoate was formed in a yield of 1.4%, and 2-naphthyl 2,2-difluorododecanoate was formed in a yield of 39%. The structural characteristics of the products are as follows. 2-Naphthyl 2-fluorododecanoate 19F-NMR (deuterated acetone): -190.2(m), 2-naphthyl 2,2-difluorododecanoate 19F-NMR (deuterated acetone): -103.9(t, J=17.0 Hz).

### [Example 17]

A solution of 0.50 g of 6-methyl-4-phenyl-2-chromanone, 2.65 g of N-fluorobenzenesulfonimide, 22 ml of THF and 6.5 ml of toluene, was cooled to -100°C, and 7.34 ml of a 1.0M THF solution of potassium hexamethyldisilazide was added. After raising the temperature to room temperature, an aqueous citric acid solution was added to terminate the reaction, followed by extraction with ethyl acetate, and the solvent was removed. 0.87 g of the obtained product was quantitatively analyzed by 19F NMR, whereby it was confirmed that 3,3-difluoro-6-methyl-4-phenyl-2-chromanone was formed in a yield of 25%. No formation of a monofluoro product was detected. The structural characteristics of the 3,3-difluoro-6-methyl-4-phenyl-2-chromanone are as follows. 19F-NMR (deuterated acetone): -96.5(bs).

As shown in Table 1, according to the production method of the present invention, the difluoro product can be obtained without forming an insoluble by-product. Further, as is evident from the comparison of Examples 2 and 3, the yield can further be improved by decomposing the electrophilic fluorinating agent.

The present invention is useful for producing a difluoro lactone compound selectively and in a high yield without forming a hardly soluble by-product.

## Claims

1. A method for producing a difluoro lactone compound represented by the following formula (4), which comprises fluorinating a lactone compound represented by the following formula (3) by reacting it with an electrophilic fluorinating agent in the presence of potassium hexamethyldisilazide and in the absence of a metal compound reactant: 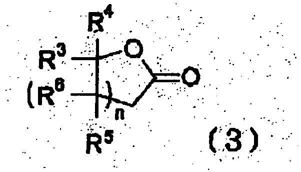 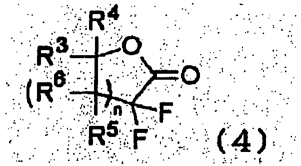 wherein each of R³, R⁴, R⁵ and R⁶ which are independent of one another, is a monovalent group selected from the group consisting of a hydrogen atom, a halogen atom, a protected hydroxy group, a protected amino group, a protected carboxy group and a C₁₋₂₀ hydrocarbon group which may have a substituent, or adjacent two among R³, R⁴, R⁵ and R⁶ are bonded to form a C₂₋₆ alkylene group which may have a substituent and other than the two among R³, R⁴, R⁵ and R⁶ are, each independently, the above monovalent group, and n is an integer of from 1 to 4.

2. The method according to Claim 1, wherein after conducting the fluorination reaction, a compound to decompose the remaining electrophilic fluorinating agent is added.

3. The method according to Claim 2, wherein the compound to decompose the electrophilic fluorinating agent is an amine or a halogen ion salt.

4. The method according to any one of Claims 1 to 3, wherein the electrophilic fluorinating agent is an electrophilic fluorinating agent selected from the group consisting of N-fluoro sulfonamides and N-fluoro sulfonimides.

5. The method according to any one of Claims 1 to 4, wherein the reaction is conducted at from -120°C to -50°C.

6. The method according to any one of Claims 1 to 5, wherein the ratio represented by the number of equivalent of the electrophilic fluorinating agent/the number of moles of the lactone compound represented by the formula (3) is from 1.6 to 12.

7. The method according to any one of Claims 1 to 6, wherein the ratio represented by the number of equivalent of potassium hexamethyldisilazide/the number of equivalent of the electrophilic fluorinating agent is from 0.5 to 2.0.

8. The method according to any one of Claims 1 to 7, wherein the lactone compound represented by the formula (3) is a lactone compound represented by the following formula (5): 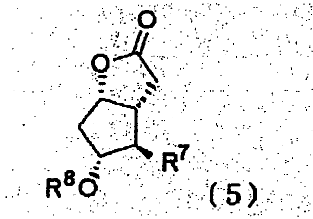 wherein R⁷ is a C₁₋₁₄ hydrocarbon group which may have a substituent, and R⁸ is a hydrogen atom or a protective group.

9. The method according to any one of Claims 1 to 7, wherein the lactone compound represented by the formula (3) is a compound represented by the following formula (9): 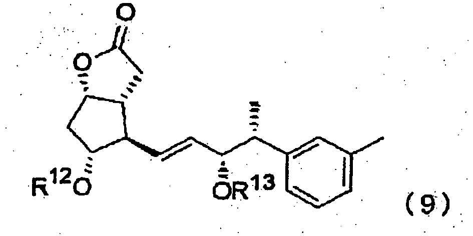 wherein each of R¹² and R¹³ which are independent of each other, is a tetrahydropyranyl group, a benzoyl group, a p-phenylbenzoyl group or a SiX₃ group, wherein X is an alkyl group, an aryl group, an aralkyl group or a heterocyclic group.

10. A method for producing a compound represented by the following formula (11), which comprises obtaining a difluoro lactone compound by the method as defined in Claim 9, and further reacting the difluoro lactone compound with (4-(1H-tetrazol-5-yl)butyl)triphenyl phosphonium bromide: 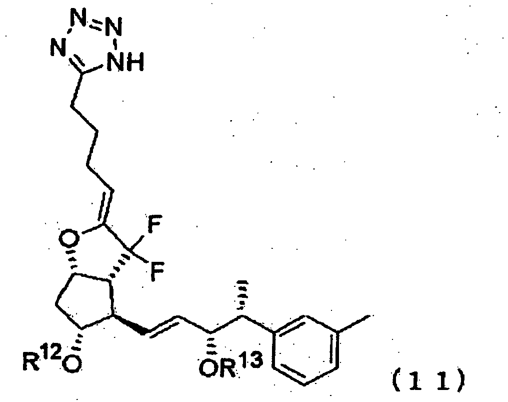 wherein each of R¹² and R¹³ which are independent of each other, is a tetrahydropyranyl group, a benzoyl group, a p-phenylbenzoyl group or a SiX₃ group, wherein X is an alkyl group, an aryl group, an aralkyl group or a heterocyclic group.

11. A method for producing a compound represented by the following formula (12), which comprises obtaining a compound represented by the formula (11) by the method as defined in Claim 10, and further eliminating R¹² and R¹³ of the compound represented by the formula (11) for substitution by hydrogen atoms.

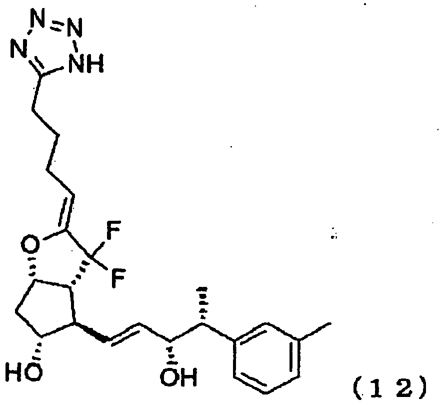

## Patentansprüche

1. Verfahren zu Herstellung einer Difluorlactonverbindung, dargestellt durch die folgende Formel (4), welches das Fluorieren einer Lactonverbindung, dargestellt durch die folgende Formel (3), durch das Umsetzen derer mit einem elektrophilen Fluorierungsmittel in der Gegenwart von Kaliumhexamethyldisilazid und in der Abwesenheit eines Metallverbindungsreaktanten umfasst: 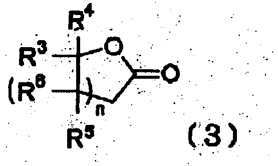 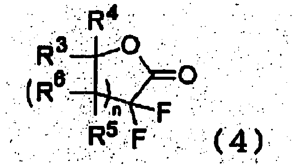 wobei jedes von R³, R⁴, R⁵ und R⁶, welche unabhängig voneinander sind, eine einwertige Gruppe, ausgewählt aus der Gruppe, bestehend aus einem Wasserstoffatom, einem Halogenatom, einer geschützten Hydroxygruppe, einer geschützten Aminogruppe, einer geschützten Carboxygruppe und einer C₁₋₂₀ Kohlenwasserstoffgruppe, welche einen Substituenten aufweisen kann, ist oder benachbarte zwei unter R³, R⁴, R⁵ und R⁶ verbunden sind, um eine C₂₋₆ Alkylengruppe zu bilden, die einen Substituenten aufweisen kann, und andere als die zwei unter R³, R⁴, R⁵ und R⁶ jeweils unabhängig die vorstehende einwertige Gruppe sind und n eine ganze Zahl von 1 bis 4 ist.

2. Verfahren nach Anspruch 1, wobei nach dem Durchführen der Fluorierungsreaktion eine Verbindung zugegeben wird, um das verbleibende elektrophile Fluorierungsmittel zu zersetzen.

3. Verfahren nach Anspruch 2, wobei die Verbindung, um das elektrophile Fluorierungsmittel zu zersetzen, ein Amin oder ein Halogenionensalz ist.

4. Verfahren nach einem der Ansprüche 1 bis 3, wobei das elektrophile Fluorierungsmittel ein elektrophiles Fluorierungsmittel, ausgewählt aus der Gruppe, bestehend aus N-Fluorsulfonamiden und N-Fluorsulfonimiden, ist.

5. Verfahren nach einem der Ansprüche 1 bis 4, wobei die Reaktion bei einer Temperatur von -120°C bis -50°C durchgeführt wird.

6. Verfahren nach einem der Ansprüche 1 bis 5, wobei das Verhältnis, dargestellt durch die Anzahl von Äquivalenten des elektrophilen Fluorierungsmittels/die Anzahl von Molen der Lactonverbindung, dargestellt durch die Formel (3), von 1,6 bis 12 beträgt.

7. Verfahren nach einem der Ansprüche 1 bis 6, wobei das Verhältnis, dargestellt durch die Anzahl von Äquivalenten von Kaliumhexamethyldisilazid/die Anzahl von Äquivalenten des elektrophilen Fluorierungsmittels, von 0,5 bis 2,0 beträgt.

8. Verfahren nach einem der Ansprüche 1 bis 7, wobei die Lactonverbindung, dargestellt durch die Formel (3), eine Lactonverbindung, dargestellt durch die folgende Formel (5), ist: 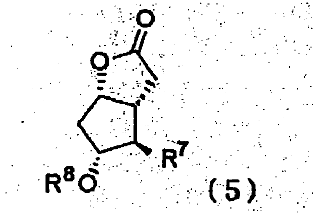 wobei R⁷ eine C₁₋₁₄ Kohlenwasserstoffgruppe ist, die einen Substituenten aufweisen kann, und R⁸ ein Wasserstoffatom oder eine Schutzgruppe ist.

9. Verfahren nach einem der Ansprüche 1 bis 7, wobei die Lactonverbindung, dargestellt durch die Formel (3), eine Verbindung, dargestellt durch die folgende Formel (9), ist: 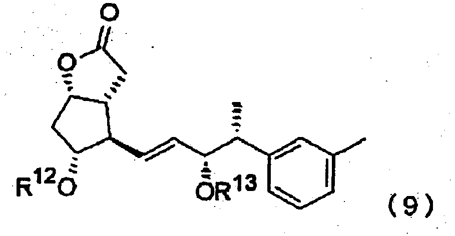 wobei jedes von R¹² und R¹³, die unabhängig voneinander sind, eine Tetrahydropyranylgruppe, eine Benzoylgruppe, eine p-Phenylbenzoylgruppe oder eine SiX₃ Gruppe ist, wobei X eine Alkylgruppe, eine Arylgruppe, eine Aralkylgruppe oder eine heterocyclische Gruppe ist.

10. Verfahren zur Herstellung einer Verbindung, dargestellt durch die folgende Formel (11), welches das Erhalten einer Difluorlactonverbindung durch das Verfahren nach Anspruch 9 und weiter das Umsetzen der Difluorlactonverbindung mit (4-(1H-Tetrazol-5-yl)butyl)triphenylphosphoniumbromid umfasst: 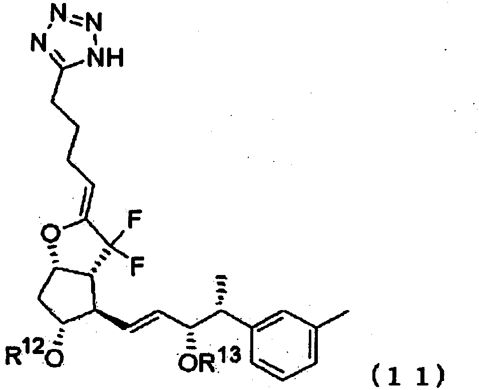 wobei jedes von R¹² und R¹³, die unabhängig voneinander sind, eine Tetrahydropyranylgruppe, eine Benzoylgruppe, eine p-Phenylbenzoylgruppe oder eine SiX₃ Gruppe ist, wobei X eine Alkylgruppe, eine Arylgruppe, eine Aralkylgruppe oder eine heterocyclische Gruppe ist.

11. Verfahren zur Herstellung einer Verbindung, dargestellt durch die folgende Formel (12), welches das Erhalten einer Verbindung, dargestellt durch die Formel (11), durch das Verfahren nach Anspruch 10 und weiter das Entfernen von R¹² und R¹³ aus der Verbindung, dargestellt durch die Formel (11), für den Austausch durch Wasserstoffatome umfasst.

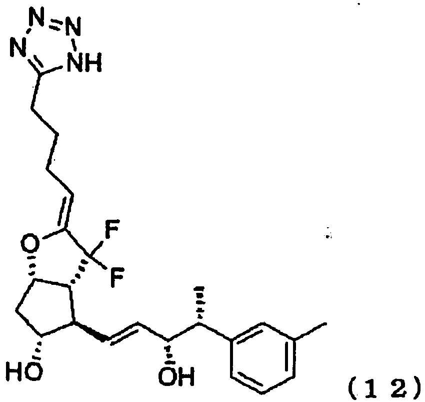

## Revendications

1. Procédé de production d'un composé lactone difluoré représenté par la formule (4) suivante, qui comprend la fluoration d'un composé lactone représenté par la formule (3) suivante en le faisant réagir avec un agent de fluoration électrophile en présence d'hexaméthyldisilazoture de potassium et en l'absence d'un réactif composé métallique : 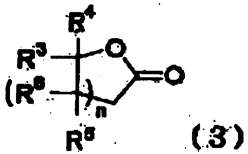 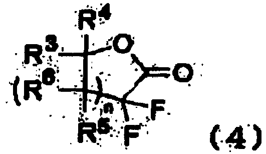 dans lequel chacun de R³, R⁴, R⁵ et R⁶ qui sont indépendants les uns des autres, est un groupe monovalent choisi dans le groupe constitué par un atome d' hydrogène, un atome d'halogène, un groupe hydroxy protégé, un groupe amino protégé, un groupe carboxy protégé et un groupe hydrocarbure en C₁ à ₂₀ qui peut comporter un substituant, ou deux éléments adjacents parmi R³, R⁴, R⁵ et R⁶ sont liés pour former un groupe alkylène en C₂ à ₆ qui peut comporter un substituant et deux autres parmi R³, R⁴, R⁵ et R⁶ sont, chacun indépendamment, le groupe monovalent ci-dessus, et n est un nombre entier de 1 à 4.

2. Procédé selon la revendication 1, dans lequel après réalisation de la réaction de fluoration, un composé pour décomposer l'agent de fluoration électrophile restant est ajouté.

3. Procédé selon la revendication 2, dans lequel le composé pour décomposer l'agent de fluoration électrophile est une amine ou un sel d'ion halogène.

4. Procédé selon l'une quelconque des revendications 1 à 3, dans lequel l'agent de fluoration électrophile est un agent de fluoration électrophile choisi dans le groupe constitué par les N-fluoro sulfonamides et les N-fluoro sulfonimides.

5. Procédé selon l'une quelconque des revendications 1 à 4, dans lequel la réaction est réalisée de -120 °C à -50 °C.

6. Procédé selon l'une quelconque des revendications 1 à 5, dans lequel le rapport représenté par le nombre d'équivalents de l'agent de fluoration électrophile/le nombre de moles du composé lactone représenté par la formule (3) est de 1,6 à 12.

7. Procédé selon l'une quelconque des revendications 1 à 6, dans lequel le rapport représenté par le nombre d'équivalents d'hexaméthyldisilazoture de potassium/le nombre d'équivalents de l'agent de fluoration électrophile est de 0,5 à 2,0.

8. Procédé selon l'une quelconque des revendications 1 à 7, dans lequel le composé lactone représenté par la formule (3) est un composé lactone représenté par la formule (5) suivante : 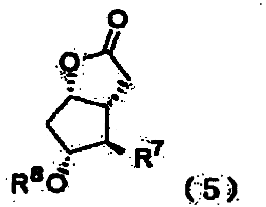 dans lequel R⁷ est un groupe hydrocarbure en C₁ à ₁₄ qui peut comporter un substituant, et R⁸ est un atome d'hydrogène ou un groupe protecteur.

9. Procédé selon l'une quelconque des revendications 1 à 7, dans lequel le composé lactone représenté par la formule (3) est un composé représenté par la formule (9) suivante : 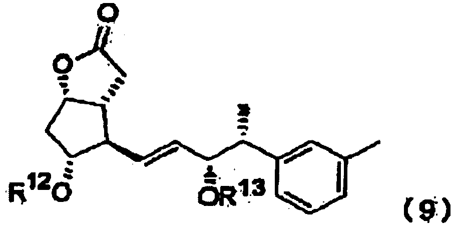 dans lequel chacun de R¹² et R¹³ qui sont indépendants l'un de l'autre, est un groupe tétrahydropyranyle, un groupe benzoyle, un groupe p-phénylbenzoyle ou un groupe SiX₃, dans lequel X est un groupe alkyle, un groupe aryle, un groupe aralkyle ou un groupe hétérocyclique.

10. Procédé de production d'un composé représenté par la formule (11) suivante, qui comprend l'obtention d'un composé lactone difluoré par le procédé tel que défini dans la revendication 9, et en outre la réaction du composé lactone difluoré avec du bromure de (4-(1H-tétrazol-5-yl)butyl)triphényl phosphonium : 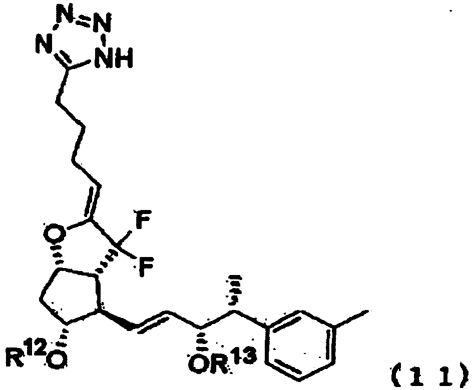 dans lequel chacun de R¹² et R¹³ qui sont indépendants l'un de l'autre, est un groupe tétrahydropyranyle, un groupe benzoyle, un groupe p-phénylbenzoyle ou un groupe SiX₃, dans lequel X est un groupe alkyle, un groupe aryle, un groupe aralkyle ou un groupe hétérocyclique.

11. Procédé de production d'un composé représenté par la formule (12) suivante, qui comprend l'obtention d'un composé représenté par la formule (11) par le procédé tel que défini dans la revendication 10, et en outre l'élimination de R¹² et R¹³ du composé représenté par la formule (11) pour substitution par des atomes d'hydrogène.
